# EUROPÄISCHE PATENTSCHRIFT

(11) **EP 1 320 339 B1**
(45) Veröffentlichungstag und Bekanntmachung des Hinweises auf die Patenterteilung: **19.01.2005**
(21) Anmeldenummer: 00960279.8
(22) Anmeldetag: 25.09.2000
(51) Int. Cl.: A61F 11/00

(54) **OTOPLASTIK UND VERFAHREN ZUR FERTIGUNG EINER OTOPLASTIK**
OTOPLASTY AND METHOD FOR PRODUCING AN OTOPLASTY
OTOPLASTIQUE ET PROCEDE DE FABRICATION D'UN OTOPLASTIQUE

(43) Veröffentlichungstag der Anmeldung: 25.06.2003
(73) Patentinhaber: PHONAK AG, 8712 Stäfa (CH)
(72) Erfinder: WIDMER, Christoph, CH-8712 Stäfa (CH); HESSEL, Hans, CH-8121 Benglen (CH); WEIDMANN, Markus, CH-8493 Saland (CH)
(74) Vertreter: Troesch, Jacques J., Dr. sc. nat.
(86) Internationale Anmeldenummer: PCT/CH2000/000525
(87) Internationale Veröffentlichungsnummer: WO 2002/024127

(56) Entgegenhaltungen:
- US-A- 4 834 927
- US-A- 5 295 191
- US-A- 5 321 757

## Beschreibung

Die vorliegende Erfindung betrifft eine Otoplastik nach dem Oberbegriff von Anspruch 1, ein Verfahren zur Fertigung von Otoplastiken nach demjenigen von Anspruch 5 sowie eine Verwendung der erwähnten Otoplastik bzw. des erwähnten Verfahrens nach Anspruch 7 und geht aus von der US-A-4'439'154.

Die vorliegende Erfindung geht von folgendem Problem aus:

Otoplastiken müssen in vielen Fällen auf den jeweiligen Applikationsbereich individualisiert werden. Dies trifft vor allem für Im-Ohr-Otoplastiken zu, die an die jeweilige individuelle Gehörgangform spezifisch angepasst sein müssen, wie dies insbesondere für Im-Ohr-Hörgeräte bekannt ist. Auch für andere Im-Ohr-Otoplastiken ist dies für optimalen Tragkomfort, äusserst wünschenswert, so beispielsweise für Kopfhörer, Gehörschutzeinrichtungen wie Lärmschutzeinrichtungen oder Wasserschutzeinrichtungen. Auch der Tragkomfort von Aussenohr-Otoplastiken kann gegebenenfalls verbessert werden, wenn die Otoplastik-Form individuell gestaltet wird. Insbesondere in der Fertigung derartiger Otoplastiken, besonders dann, wenn sie mit ebenso individualisierten Modulen wie insbesondere Elektronikmodulen ausgestattet werden, ergeben sich nur mit relativ grossem Aufwand zu lösende Probleme bezüglich der sicheren Nachverfolgung und der Vermeidung jeglicher Verwechslungen einerseits von Otoplastikschalen, anderseits der individuell damit auszurüstenden Module. Die erwähnte Problematik stellt sich aber sogar bei Otoplastiken, die ohne individualisierte Module, wie die erwähnten Elektronikmodule, aufgebaut werden, nämlich dadurch ,dass letztendlich die richtige individuelle Otoplastik an den richtigen Empfänger versandt werden muss.

Aus der US-A-4 439 154 ist es bekannt, an einer Dentalprothese oder an einem Hörgerät einen speziellen Träger mit Information vorzusehen oder informative Strukturen durch Einkratzen in die Prothese aufzubringen.

Ausgehend von einem Vorgehen letztgenannter Art stellt sich die vorliegende Erfindung zur Aufgabe, die informative Kennzeichnung zu vereinfachen.

Hierzu zeichnet sich die erfindungsgemässe Otoplastik nach dem Kennzeichen von Anspruch 1 aus.

Obwohl die erwähnten, erfindungsgemäss vorgesehenen Kennzeichnungsein- und/oder Ausbuchtungen auch Herstellerbezeichnungen, Materialbezeichnungen, Links-/Rechts-Ohr-Applikationshinweise, Serienummer etc. beinhalten können, kennzeichnen sie in weitaus bevorzugter Art und Weise, ggf. unter anderem, die jeweilige individuelle, für ein bestimmtes Individuum gefertigte Schale.

Um im weiteren eine visuelle oder maschinelle Erkennung der erwähnten Kennzeichnungsein- und/oder -ausbuchtungen zu erleichtern, wird vorgeschlagen, dass die Einbuchtungen und/oder Ausbuchtungen mindestens teilweise und mindestens zum Teil mit einem Material belegt sind, das anders ist als das Schalenmaterial, so vorzugsweise mit einer Farbe oder einem Lack. Dadurch kann die visuelle Erkennbarkeit massgebend erleichtert werden, aber insbesondere die maschinelle Lesbarkeit beispielsweise durch Laserabtastung und Reflexionsauswertung. Bei der erfindungsgemässen Otoplastik handelt es sich besonders bevorzugt um ein Hörgerät, dabei ein Aussenohr- oder Im-Ohr-Hörgerät, ganz besonders bevorzugt ein Im-Ohr-Hörgerät.

Das erfindungsgemässe Herstell-Verfahren zur Lösung des genannte Problemes zeichnet sich nach dem Kennzeichen von Anspruch 5 aus.

Dabei kann die weitere Fertigung, wie insbesondere die Assemblierung der Schale mit einzubauenden Modulen, wie Elektronikmodulen, Batterien etc., anhand der die Fertigung begleitende Schale individualisiert werden. Mindestens schalenseitig ist jederzeit erkenntlich, um welche individuelle Schale es sich handelt, womit sogar bei manueller Fertigung bzw. Assemblierung die jeweils richtigen Module eingesetzt werden können. Eine besonders bevorzugte Ausführungsform des erfindungsgemässen Verfahrens, welches die erfindungsgemäss individualisierende Kennzeichnung voll ausnützt, ergibt sich dadurch, dass mindestens ein Teil der der Schalenfertigung nachgestaffelten Fertigungsschritte durch maschinelle Erkennung der Kennzeichnung automatisiert wird. Das erfindungsgemässe Verfahren eignet sich insbesondere in seiner Verwendung für die Fertigung von Im-Ohr- oder Aussenohr-Hörgeräten, dabei insbesondere für Im-Ohr-Hörgeräte, bei denen ganz besonders auf die individuelle Fertigung und die Vermeidung jeglicher Verwechslungen zu achten ist, basierend auf der stark individuellen Gehörgangform. Otoplastiken, bei denen die vorliegende Erfindung realisiert werden kann und in bevorzugten Ausführungsformen werden anschliessend anhand von Figuren dargestellt. Diese zeigen beispielsweise:
- Fig. 1: ein vereinfachtes Schema einer Fertigungsanlage für die Optimierung industrieller Fertigung von Otoplastiken;
- Fig. 2: in einer Darstellung analog zu derjenigen von Fig. 1, eine weitere Anlagenkonzeption;
- Fig. 3: in Darstellung analog zu denjenigen der Figuren 1 und 2, eine noch weitere Anlagenkonzeption;
- Fig. 4: schematisch ein Im-Ohr-Hörgerät mit auf bekannte Art und Weise aufgesetzter Cerumen-Schutzkappe;
- Fig. 5: in Darstellung analog zu Fig. 4, ein mit Cerumen-Schutzkappe gefertigtes Im-Ohr-Hörgerät;
- Fig. 6: ein Im-Ohr-Hörgerät mit einer auf bekannte Art und Weise eingearbeiteten Belüftungsnut;
- Fig. 7(a) bis (f): perspektivisch dargestellte Ausschnitte von Otoplastik-Schalenoberflächen, mit Belüftungsnuten;
- Fig. 8: anhand eines schematischen Ausschnittes einer Otoplastik-Oberfläche, eine Belüftungsnut mit entlang ihrer Längsausdehnung variierendem Querschnitt bzw. variierender Querschnittsform;
- Fig. 9: schematisch eine Im-Ohr-Otoplastik mit verlängerter Belüftungsnut;
- Fig. 10: in Darstellung analog zu Fig. 9, eine Im-Ohr-Otoplastik mit mehreren Belüftungsnuten;
- Fig. 11 (a) bis (e): Ausschnitte von Otoplastikschalen mit Belüftungskanälen verschiedener Querschnittsformen und Dimensionen;
- Fig. 12: in einer Darstellung analog zu derjenigen von.Fig. 8, ein Belüftungskanal in einer Otoplastikschale mit entlang seiner Längsausdehnung variierender Querschnittsform bzw. variierender Querschnittsfläche;
- Fig. 13: in Analogie zur Darstellung von Fig. 9, schematisch eine Im-Ohr-Otoplastik mit eingearbeitetem, verlängerten Belüftungskanal;
- Fig. 14: in Darstellung analog zu Fig. 10, eine Im-Ohr-Otoplastik mit mehreren Belüftungskanälen;
- Fig. 15: schematisch eine Länggsschnittdarstellung einer Im-Ohr-Otoplastik mit gerippter Innenfläche;
- Fig. 16: einen Ausschnitt der Otoplastik gemäss Fig. 15 im Querschnitt, wobei die Rippen unterschiedliche Querschnittsflächen aufweisen;
- Fig. 17: perspektivisch den Ausschnitt einer Otoplastikschale mit Innenrippung nach Fig. 15 oder 16, wobei die Rippen entlang ihrer Längsausdehnung unterschiedliche Querschnittsformen und Dimensionen aufweisen;
- Fig. 18: in Darstellung analog zu Fig. 15, eine Im-Ohr-Otoplastik mit Aussenrippung;
- Fig. 19: schematisch einen Ausschnitt aus einer gemäss Fig. 18 gerippten Otoplastikschale mit Rippen unterschiedlicher Querschnittsflächen;
- Fig. 20: schematisch einen Querschnitt durch eine Otoplastik mit Aussenrippung, ggf. Innenrippung, und mindestens teilweise Füllmaterial-gefülltem Innenraum;
- Fig. 21: schematisch einen Längsschnitt-Ausschnitt einer Otoplastikschale mit biege- und stauchflexibler Partie;
- Fig. 22: schematisch im Längsschnitt, eine Im-Ohr-Otoplastik mit Aufnahmeraum für ein Elektronikmodul;
- Fig. 23: die Otoplastik nach Fig. 22 bei ihrem Aufstülpen über ein Elektronikmodul;
- Fig. 24: perspektivisch und schematisch, eine Im-Ohr-Otoplastik, wie insbesondere ein Im-Ohr-Hörgerät, mit zweiteiliger, separierbarer und assemblierbarer Otoplastikschale;
- Fig. 25: ausschnittsweise und schematisch, die Integration von akustischen Leitern und Anpassgliedern zu einem akustisch/elektrischen oder elektrisch/akustischen Wandler, in einer Otoplastik;
- Fig. 26: in Darstellung analog zu derjenigen von Fig. 25, die Anordnung zweier oder mehrerer akustischer Leiter in der Schale einer Otoplastikschale, und
- Fig. 27: anhand eines vereinfachten Signalfluss/Funktionsblockdiagrammes, ein Vorgehen bzw. eine Anordnung zu dessen Ausführung, bei dem bzw. der die Dynamik des Applikationsbereiches einer Otoplastik für deren Formgebung berücksichtigt wird.

Die im Anschluss an das Fertigungsverfahren beschriebenen Ausführungsformen von Otoplastiken werden vorzugsweise alle mit diesem beschriebenen Fertigungsverfahren hergestellt.

### Definition

Wir verstehen unter einer Otoplastik eine Einrichtung, die unmittelbar ausserhalb der Ohrmuschel und/oder an der Ohrmuschel und/oder im Gehörgang appliziert wird. Dazu gehören Aussenohr-Hörgeräte, Im-Ohr-Hörgeräte, Kopfhörer, Lärmschutz- und Wasserschutzeinsätze etc.

### 1. Fertigungsverfahren

Das Fertigungsverfahren, welches bevorzugterweise eingesetzt wird, die nachfolgend im einzelnen beschriebenen Otoplastiken zu fertigen, beruht darauf, die Form eines individuellen Applikationsbereiches für eine beabsichtigte Otoplastik dreidimensional zu digitalisieren, dann die Otoplastik oder deren Schale durch ein additives Aufbauverfahren zu erstellen. Additive Aufbauverfahren sind auch unter dem Begriff "Rapid Prototyping" bekannt. Bezüglich derartiger im schnellen Prototypenbau bereits eingesetzter additiver Verfahren wird z.B. verwiesen auf:
- http://ltk.hut.fi/∼koukka/RP/rptree.html (1)
oder auf
- Wohlers Report 2000, Rapid Prototyping & Tooling State of the industry (2)

Aus der Gruppe dieser für den schnellen Prototypenbau heute bekannten additiven Verfahren ergibt sich, dass Lasersintern, Laser- bzw. Stereolithographie oder das Thermojetverfahren sich besonders gut eignen, Otoplastiken bzw. deren Schalen aufzubauen und dabei insbesondere die nachfolgend beschriebenen speziellen Ausführungsformen. Deshalb sei, nur kurz zusammenfassend, auf Spezifikationen dieser bevorzugt eingesetzten additiven Aufbauverfahren eingegangen:
- Lasersintern: Auf einem Pulverbett wird, beispielsweise mittels eines Rollers, Heissschmelzpulver in einer dünnen Schicht aufgetragen. Mittels eines Laserstrahls wird die Pulverschicht verfestigt, wobei der Laserstrahl u.a. entsprechend einer Schnittschicht der Otoplastik bzw. Otoplastikschale mittels der 3D-Forminformation des individuellen Applikationsbereiches angesteuert wird. Es entsteht in dem im übrigen losen Pulver eine verfestigte Schnittschicht der Otoplastik bzw. deren Schale. Diese wird aus der Pulververlegeebene abgesenkt und darüber eine neue Pulverschicht aufgebracht, diese wiederum einer Schnittschicht entsprechend laserverfestigt, etc.
- Laser- bzw. Stereolithographie: Eine erste Schnittschicht einer Otoplastik bzw. einer Otoplastikschale wird mittels UV-Laser an der Oberfläche flüssigen Fotopolymers verfestigt. Die verfestigte Schicht wird abgesenkt und wird wieder von Flüssigpolymer bedeckt. Mittels des erwähnten UV-Lasers wird, auf der bereits verfestigten Schicht, die zweite Schnittschicht der Otoplastik bzw. deren Schale verfestigt. Wiederum erfolgt die Laserpositionssteuerung u.a. mittels der 3D-Daten bzw. Information des individuellen, vorgängig erfassten Applikationsbereiches.
- Thermojetverfahren: Die Konturbildung entsprechend einer Schnittschicht der Otoplastik bzw. der Otoplastikschale wird ähnlich wie bei einem Tintenstrahldrucker durch Flüssigauftrag u.a. gemäss der digitalisierten 3D-Forminformation, insbesondere auch des individuellen Applikationsbereiches vorgenommen. Danach wird die abgelegte Schnitt-"Zeichnung" verfestigt. Wiederum wird gemäss dem Prinzip der additiven Aufbauverfahren Schicht um Schicht zum Aufbau der Otoplastik bzw. deren Schale abgelegt.
   Es kann bezüglich additiver Aufbauverfahren und der obgenannten bevorzugten auf folgende weitere Veröffentlichungen hingewiesen werden:
   - http://www.padtinc.com/srv_rpm_sls.html (3)
   - "Selective Laser Sintering (SLS) of Ceramics", Muskesh Agarwala et al., presented at the Solid Freeform Fabrication Symposium, Austin, TX, August 1999, (4)
   - http://www.caip.rutgers.edu/RP_Library/process.html (5)
   - http://www.biba.uni-bremen.de/groups/rp/lom.html bzw.
   - http://www.biba.uni-bremen.de/groups/rp/rp_intro.html (6)
   - Donald Klosterman et al., "Direct Fabrication of Polymer Composite Structures with Curved LOM", Solid Freeform Fabrication Symposium, University of Texas at Austin, August 1999, (7)
   - http://lff.me.utexas.edu/sls.html (8)
   - http://www.padtinc.com/srv_rpm_sla.html (9)
   - http://www.cs.hut.fi/∼ado/rp/rp.html (10)

Grundsätzlich wird somit bei additiven Aufbauverfahren jeweils eine dünne Materialschicht auf einer Fläche abgelegt, sei dies wie beim Lasersintern oder der Stereolithographie noch ganzflächig, sei dies wie beim Thermojetverfahren bereits in der Kontur eines Schnittes der im Aufbau begriffenen Otoplastik bzw. deren Schale. Daraufhin wird die erwünschte Schnittform stabilisiert bzw. verfestigt.

Ist eine Schicht verfestigt, so wird darüber eine neue Schicht wie beschrieben abgelegt und diese wiederum verfestigt und mit der darunter liegenden, schon fertig gestellten Schicht verbunden. So wird Schicht um Schicht die Qtoplastik bzw. deren Schale erstellt, durch additives Schicht-um-Schicht-Auftragen.

Für die industrielle Fertigung wird bevorzugterweise jeweils nicht nur die Schnittschicht für eine individuelle Otoplastik bzw. deren Schale abgelegt bzw. verfestigt, sondern gleichzeitig mehrere je individuelle. Bei Lasersintern verfestigt z.B. der eine Laser, üblicherweise spiegelgesteuert, hintereinander die Schnittschichten mehrerer Otoplastiken bzw. deren Schalen, bevor alle verfestigten Schnittschichten gemeinsam abgesenkt werden. Daraufhin, nach Ablegen einer neuen Pulverschicht über alle bereits verfestigten und abgesenkten Schnittschichten, erfolgt wiederum die Bildung der mehreren weiteren Schnittschichten. Trotz dieser parallelen Fertigung werden die jeweiligen Otoplastiken bzw. deren Schalen, digital gesteuert, individuell gefertigt.

Dabei wird zur Verfestigung der mehreren Schnittschichten entweder ein einziger Laserstrahl eingesetzt und/oder es werden mehr als ein Strahl parallel betrieben und angesteuert.

Eine Alternative zu diesem Vorgehen besteht darin, jeweils mit einem Laser eine Schnittschicht zu verfestigen, während gleichzeitig für die Bildung einer weiteren Otoplastik bzw. Otoplastikschale die Pulverschicht abgelegt wird. Danach wird der nämliche Laser die bereitete Pulverschicht entsprechend der Schnittschicht für die weitere Plastik verfestigen, während die davor verfestigte Schicht abgesenkt und dort eine neue Pulverschicht abgelegt wird. Der Laser arbeitet dann intermittierend zwischen zwei oder mehreren im Aufbau begriffenen Otoplastiken bzw. Otoplastikschalen, wobei die durch die Pulverablage bei der Bildung einer der Schalen entstehende Lasereinsatz-Totzeit für die Verfestigung einer Schnittschicht einer anderen im Aufbau begriffenen Otoplastik ausgenützt wird.

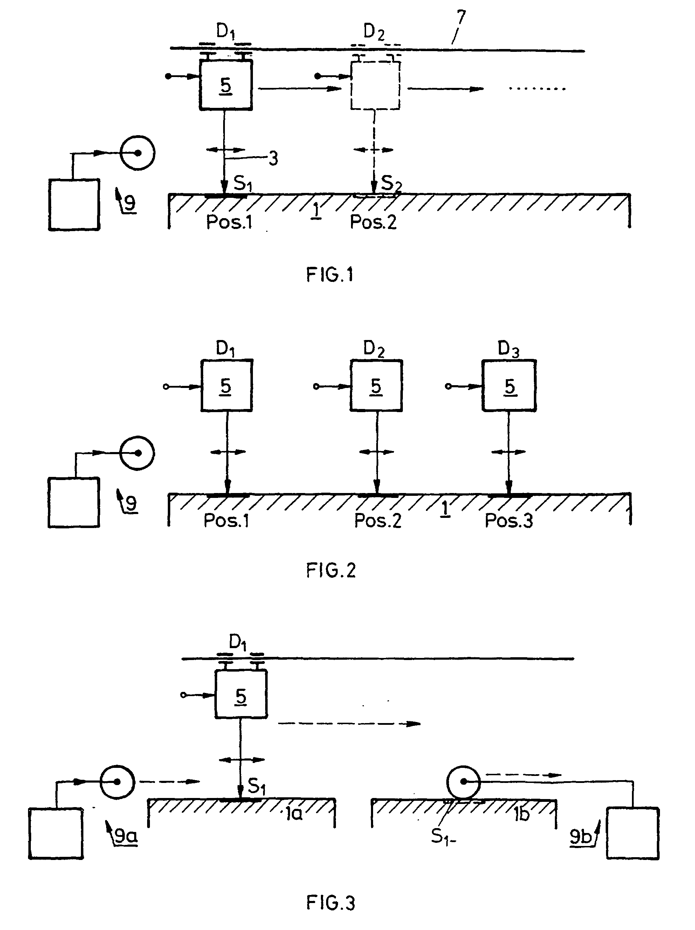

In Fig. 1 ist schematisch dargestellt, wie, in einer Ausführungsvariante, mittels Lasersintern oder Laser- bzw. Stereolithographie mehrere Otoplastiken bzw. deren Schalen in einem Parallelprozess industriell gefertigt werden. Über dem Materialbett 1 für Pulver oder Flüssigmedium ist der Laser mit Steuereinheit 5 und Strahl 3 montiert. In Position 1 verfestigt er die Schicht S₁ einer ersten Otoplastik bzw. deren Schale, angesteuert mit dem ersten individuellen Datensatz D₁. Danach wird er an einer Verschiebevorrichtung 7 in eine zweite Position verstellt, wo er mit dem individuellen Datensatz D₂ die Schicht S₂ entsprechend einer weiteren Individualkontur erstellt. Selbstverständlich können mehrere der Laser als Einheit verschoben werden und jeweils mehr als eine individuelle Otoplastikschicht gleichzeitig erstellt werden. Erst wenn die vorgesehenen Laser 5 in allen vorgesehenen Positionen die jeweiligen individuellen Schichten erstellt haben, wird mit der generell bei 9 dargestellten Pulverzuführung im Falle des Lasersintern eine neue Pulverschicht abgelegt, während (nicht dargestellt) bei der Laser- bzw. Stereolithographie die verfestigten Schichten S im Flüssigbett abgesenkt werden.

Gemäss Fig. 2 werden gleichzeitig an einem oder mehreren Flüssigkeits- bzw. Pulverbetten 1, mit mehreren gleichzeitig individuell angesteuerten Lasern 5, Schichten individueller Otoplastiken bzw. deren Schalen verfestigt. Wiederum wird mit der Pulverausgabeeinheit 9 nach Erledigung dieser verfestigungsphase und nach Stillsetzen der Laser eine neue Pulverschicht abgelegt, während im Falle der Laser- bzw. Stereolitographie die eben verfestigten Schichten bzw. bereits verfestigten Aufbauten im Flüssigbett abgesenkt werden.

Gemäss Fig. 3 verfestigt Laser 5 am einen Pulver- bzw. Flüssigbett 1a die Schicht S₁, um danach zum Bett 1b überzuwechseln (gestrichelt), woran während der Verfestigungsphase am Bett 1a die Pulverauftragsvorrichtung 9b über einer vorgängig verfestigten Schicht S₁₋ Pulver abträgt bzw., bei der Laser- oder Stereolithographie, die Schicht S₁₋ abgesenkt wird. Erst wenn der Laser 5 am Bett 1b aktiv wird, erfolgt mit der Pulverausgabevorrichtung 9a das Ablegen einer neuerlichen Pulverschicht über der eben verfestigten Schicht S₁ am Bett 1a bzw. erfolgt Absenken der Schicht S₁ im Flüssigbett 1a.

Beim Einsatz der Thermojetverfahren und zur analogen Produktivitätserhöhung werden gleichzeitig Schnittschichten von mehr als einer Otoplastik bzw. deren Schalen abgelegt, praktisch in einem Zeichnungszug durch einen Auftragungskopf oder, parallel, durch mehrere.

Durch das dargestellte Verfahren ist es möglich, höchst komplexe Formen an Otoplastiken bzw. deren Schalen zu realisieren, und zwar sowohl was ihre Aussenformung mit individueller Anpassung an den Applikationsbereich anbelangt als auch was, bei einer Schale, deren Innenformung anbelangt. Überhänge, Ein- und Aussprünge können ohne weiteres realisiert werden.

Im weiteren sind Materialien für additive Aufbauverfahren bekannt, welche zu einer gummielastischen und doch formstabilen Schale geformt werden können, die, falls erwünscht, lokal unterschiedlich bis zu äusserst dünnwandig und trotzdem reissstabil realisiert werden kann.

In einer heute bevorzugten Ausführungsform wird die Digitalisierung des individuellen Applikationsbereiches, insbesondere des Applikationsbereiches für ein Hörgerät, dabei insbesondere Im-Ohr-Höhrgerät, bei einer spezialisierten Institution, im letzterwähnten Falle beim Audiologen, vorgenommen. Die dort aufgenommene Individualform, als digitale 3D-Information, wird, insbesondere im Zusammenhang mit Hörgeräten, an ein Produktionszentrum übermitteln, sei dies durch Übersendung eines Datenträgers, sei dies durch Internetverbindung etc. Im Produktionszentrum wird, insbesondere unter Einsatz der oben erwähnten Verfahren, die Otoplastik bzw. deren Schale, im betrachteten Fall also die Im-Ohr-Hörgeräteschale, individuell geformt. Bevorzugterweise wird auch dort die Fertigassemblierung des Hörgerätes mit den funktionellen Baugruppen vorgenommen.

Aufgrund der Tatsache, dass, wie erwähnt, die eingesetzten Thermoplastmaterialien im allgemeinen zu einer relativ elastischen, sich anschmiegenden Aussenform führen, ist auch die Formgebung bezüglich Druckstellen bei Otoplastiken bzw. deren Schalen weit weniger kritisch, als dies bis anhin der Fall war, was insbesondere für Im-Ohr-Otoplastiken von ausschlaggebender Bedeutung ist. So können Im-Ohr-Otoplastiken beispielsweise als Gehörschutzeinrichtungen, Kopfhörer, Wasserschutzeinrichtungen, aber insbesondere auch für Im-Ohr-Hörgeräte, ähnlich gummielastischen Pfropfen eingesetzt werden, und es schmiegt sich deren Oberfläche optimal an den Applikationsbereich, den Gehörgang, an. Ohne weiteres ist dabei das Einarbeiten eines oder mehrerer Belüftungskanäle in die Im-Ohr-Otoplastik möglich, um beim resultierenden, möglicherweise relativ dichten Sitz der Otoplastik im Gehörgang eine unbeeinträchtigte Belüftung zum Trommelfell sicherzustellen. Dabei kann mit den individuellen 3D-Daten des Applikationsbereiches bei der Fertigung auch der Innenraum der Plastik optimiert und optimal genutzt werden, auch individuell bezüglich der ggf. aufzunehmenden individuellen Aggregat-Konstellation wie bei einem Hörgerät.

Insbesondere bei Otoplastiken in der Form von Hörgeräten kann durch die zentrale Fertigung ihrer Schalen eine zentrale Abspeicherung und Verwaltung von Individualdaten, sowohl bezüglich des individuellen Applikationsbereiches, wie auch der individuellen Funktionsteile und ihrer Einstellungen, vorgenommen werden. Muss, aus welchen Gründen auch immer, eine Schale ersetzt werden, so kann sie ohne weiteres durch Abruf der individuellen Datensätze neuerlich gefertigt werden, ohne dass eine mühselige Neuanpassung - wie bis anhing - notwendig wäre.

Aufgrund der Tatsache, dass die für die Fertigung von Otoplastiken beschriebenen Verfahren, allerdings lediglich für den Prototypenbau, bekannt sind und in der Literatur beschrieben sind, erübrigt sich an dieser Stelle eine Wiedergabe aller technischen Einzelheiten bezüglich dieser Verfahren.

Jedenfalls ergeben sich überraschenderweise aus Übernahme dieser aus dem Prototypenbau vorbekannten Technologien für die industrielle, kommerziell vertretbare Fertigung von Otoplastiken ganz wesentliche Vorteile, und zwar aus Gründen, die, an sich, im Prototypenbau nicht massgebend sind, wie z.B. Elastizität der verwendbaren thermoplastischen Materialien, der Möglichkeit, höchst dünnwandig individuell zu bauen etc.

Zusammenfassend wird es durch Einsatz der erwähnten additiven Aufbauverfahren für die Fertigung von Otoplastiken bzw. deren Schalen möglich, daran verschiedene funktionale Elemente zu integrieren, die konstruktiv bereits während der Planung der Otoplastik am Rechner vorbereitet werden und die mit dem Aufbau der Otoplastik bzw. deren Schale erzeugt werden. Typischerweise wurden derartige funktionale Elemente bisher erst nach der Fertigstellung der Otoplastik bzw. deren Schale in diese eingepasst bzw. an diese angefügt, was an materiellen Schnittstellen oder Materialinhomogenität an den Verbindungsstellen erkenntlich ist.

Für die erwähnten Otoplastiken, insbesondere mit elektronischen Einbauten, wie für Hörgeräte, dabei insbesondere für Im-Ohr-Hörgeräte, sind solche Elemente, die mit der vorgeschlagenen Technik direkt in die Otoplastikschale eingebaut werden können, beispielsweise: Aufnahmen und Halterungen für Bauteile, Cerumen-Schutzsysteme, Belüftungskanäle bei Im-Ohr-Otoplastiken, Stützelemente, die bei Im-Ohr-Otoplastiken letztere im Gehörgang haltern, wie sogenannte Krallen (englisch channel locks).

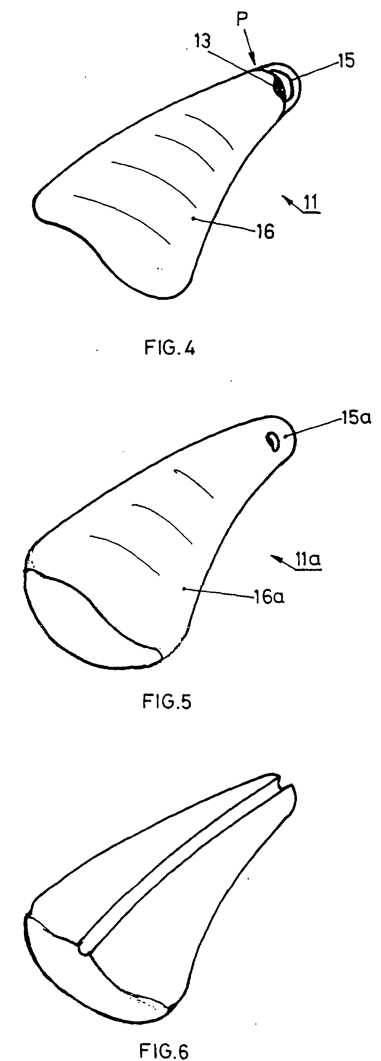

In Fig. 4 ist beispielsweise und schematisiert eine Im-Ohr-Otoplastik 11 dargestellt, beispielsweise ein Im-Ohr-Hörgerät, bei dem der akustische Ausgang 13 zum Trommelfell mittels einer Cerumen-Schutzkappe 15 geschützt ist. Diese Schutzkappe 15 wird bis anhin in der Herstellung als separater Teil auf die Schale 16 der Otoplastik 11 aufgebracht und beispielsweise durch Verkleben oder Verschweissen fixiert. Wie in Fig. 5 in gleicher Darstellung gezeigt, wird durch Einsatz der erwähnten additiven Aufbauverfahren die Cerumen-Schutzkappe 15a direkt an die Schale 16a der sonst identischen Im-Ohr-Otoplastik 11a integriert. An den in Fig. 4 mit P schematisch angedeuteten Verbindungsstellen, wo bei herkömmlichen Verfahren zwangsweise eine Material-Inhomogenität bzw. -Schnittstelle entsteht, liegt gemäss Fig. 5 keine derartige Schnittstellen vor, das Material der Schale 16a geht homogen in dasjenige der Cerumen-Schutzkappe 15a über.

Dies nur als Beispiel, wie bekannte Cerumen-Schutzsysteme und andere funktionale Elemente durch Einsatz des erwähnten Fertigungsverfahrens integral eingebaut werden können.

Es werden nachfolgend einige spezifische neuartige Otoplastiken vorgestellt:

### 2. Innenohr-Otoplastiken mit Entlüftung

Es ist bekannt, bei Im-Ohr-Otoplastiken, insbesondere bei Im-Oh-Hörgeräten, eine Belüftungsrinne auf der Aussenseite vorzusehen, wie dies schematisch in Fig. 6 dargestellt ist. Solche Belüftungsrinnen, wie sie heute eingesetzt werden, sind unter verschiedenen Aspekten keinesfalls optimiert:
- Bezüglich akustischem Verhalten: Die heute bekannten Belüftungsrinnen sind kaum an die jeweiligen akustischen Erfordernisse angepasst. So können sie kaum, bei aktiven Otoplastiken, wie z.B. bei Im-Ohr-Hörgeräten, dazu beitragen, die Rückkopplungsproblematik von elektromechanischem Ausgangswandler zu akustisch/elektrischem Eingangswandler wirksam lösen zu helfen. Auch bei passiven Im-Ohr-Otoplastiken, wie Gehörschutz-Einrichtungen, vermögen sie nicht, das erwünschte Schutzverhalten zu unterstützen und gleichzeitig die erwünschten Belüftungseigenschaften beizubehalten.
- Cerumenempfindlichkeit: Die heute eingesetzten Belüftungsrinnen in der Aussflächen von Im-Ohr-Otoplastiken sind äusserst Cerumenbildungs-empfindlich. Die Cerumenbildung vermag, je nach deren Intensität, rasch die vorgesehene Belüftungsrinnen bezüglich ihrer Belüftungseigenschaften zu beeinträchtigen, wenn nicht gar vollständig zu verstopfen.

Es werden nachfolgend für Im-Ohr-Otoplastiken, dabei insbesondere für Im-Ohr-Hörgeräte oder Gehörschutzeinrichtungen, aber auch für Otoplastiken, die nur teilweise in den Gehörgang einragen, wie Kopfhörer, Belüftungsvorkehrungen vorgeschlagen, die die obgenannten Nachteile bekannter Vorkehrungen mindestens teilweise beheben.

Hierzu werden nachfolgend Belüftungssysteme unterschieden, die
- nutenähnlich gegen die Gehörgangwandung mindestens zum Teil offen sind,
- gegen die Wandung des Gehörganges hin vollständig geschlossen sind.

### 2a) Gegen die Wandung des Gehörganges offene Belüftungssysteme

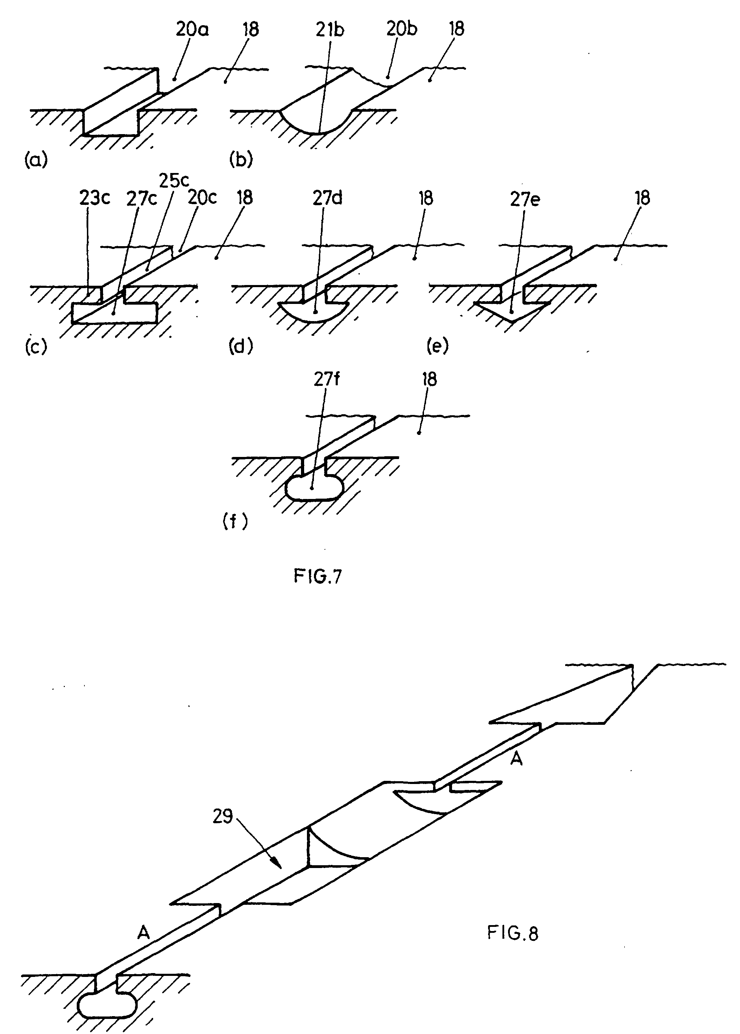

In den Fig. 7(a) bis (f) sind, anhand perspektivischer, schematischer Darstellungen von Ausschnitten der am Gehörgang anliegenden Aussenwandung 18 von im-Ohr-Otoplastiken, neuartige Belüftungsnutprofile ausschnittsweise dargestellt. Gemäss Fig. 7(a) ist das Profil der Belüftungsnut 20a rechteck- oder quadratförmig mit vorgegebenen, exakt eingehaltenen Dimensionierungsverhältnissen. Gemäss Fig. 7(b) ist das Profil der Belüftungsnut 20b Kreis- oder Ellipsen-sektorförmig, wiederum mit exakt vorgegebener Querschnittsberandungskurve 21b. Durch exakte Vorgabe und Realisierung der Querschnittsform der vorgesehenen Belüftungsnuten 20 kann bereits eine gewisse Vorhersagbarkeit und Beeinflussung der akustischen Übertragungsverhältnisse entlang dieser Nut, bei Anliegen an der Gehörgang-Innenwand, realisiert werden. Selbstverständlich ist das akustische Verhalten auch abhängig von der Länge, mit welcher sich die Nut 20 entlang der Otoplastik-Aussenwand 18 erstreckt.

In den Fig. 7(c) bis (f) sind weitere Belüftungsnutprofile dargestellt, welche zusätzlich Cerumen-geschützt sind. Das Profil der Nut 20c gemäss Fig. 7(c) ist T-förmig.

Bezüglich der weiten Nutquerschnittsfläche bei 27c bewirken die einkragenden Partien 23c und die sich daraus ergebende Verengung 25c, gegen die Wand des Gehörganges hin, bereits eine ansehnliche Cerumenschutzwirkung. Auch wenn Cerumen in die Verengung 25c eindringt und dort verhärtet, ergibt sich dadurch noch keine wesentliche Verengung oder gar Verstopfung der Belüftungsnut, die nun zum geschlossenen Belüftungskanal wird. In den Fig. 7(d) bis 7(f) ist, dem erläuterten Prinzip von Fig. 7(c) folgend, die Querschnittsform der weiten Nutpartie 27d bis 27f mit unterschiedlicher Formung ausgebildet, gemäss Fig. 7(d) kreissektorförmig bzw. entsprechend dem Sektor einer Ellipse, gemäss Fig. 7(e) dreieckförmig, gemäss Fig. 7(f) kreisförmig bzw. elliptisch.

Durch gezielte Auslegung der Nutquerschnittsfläche, wie dies nur beispielsweise anhand der Figuren 7(a) bis 7(f) dargestellt ist, lässt sich sowohl bezüglich akustischen Eigenschaften wie auch bezüglich Cerumenschutzwirkung eine bereits in starkem Mass gegenüber herkömmlichen, mehr oder weniger zufällig profilierten Belüftungsnuten verbesserte Wirkung erzielen. Dabei werden die Profile unter Berücksichtigung der erwähnten Cerumenschutzwirkung und der akustischen Wirkung vorgängig rechnerisch modelliert und exakt in die gefertigten Otoplastiken integriert. Hierzu eignen sich in ganz besonderem Umfang die oben erläuterten additiven Aufbauverfahren. Um nun weiter die akustische Wirkung der Belüftungsnut zu optimieren, können entlang der neuartigen Belüftungsnuten die unterschiedlichsten akustischen Impedanzen realisiert werden, was beispielsweise gemäss Fig. 8 in Entlüftungsnuten 29 resultiert, die, in ihrer Längsrichtung fortschreitend, unterschiedliche Profile definieren, wie sie wahlweise in Fig. 8 aus Profilen gemäss Fig. 7 zusammengestellt dargestellt sind.

Ähnlich der Auslegung passiver elektrischer Netzwerke, kann dadurch das akustische Übertragungsverhalten der am Gehörgang anliegenden Nut rechnerisch modelliert und überprüft werden, dann in die Im-Ohr-Otoplastik bzw. deren Schale integriert werden.

Gezielt können vermehrt Cerumen-geschützte Abschnitte an diesbezüglichen, ausgesetzten Partien, wie in Fig. 8 bei A dargestellt, vorgesehen werden.

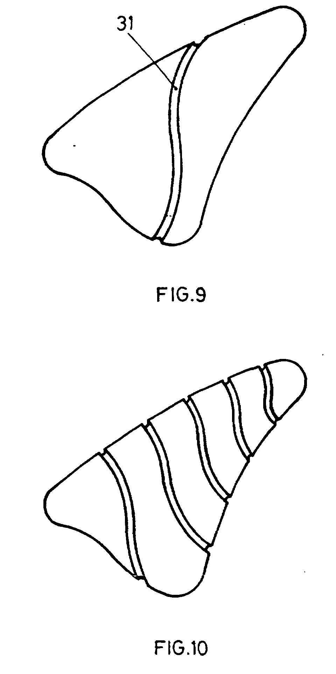

Im weiteren kann es durchaus gewünscht sein, gerade mit Blick auf die Optimierung der akustischen Verhältnisse, die vorgesehenen Belüftungsnuten länger auszubilden, als dies grundsätzlich durch die Längsausdehnung einer betrachteten Im-Ohr-Otoplastik gegeben ist. Wie in Fig. 9 dargestellt, wird dies dadurch erreicht, dass solche Nuten 31 mit Ausbildung, wie sie anhand der Fig. 7 und 8 beispielsweise dargestellt werden, in vorgegebenen Kurven entlang der Oberfläche der Otoplastik geführt werden, beispielsweise wie in Fig. 9 dargestellt, praktisch als die Otoplastik gewindeartig umschlingende Nuten. Weitere Optimierungsflexibilität wird dadurch erreicht, dass nicht nur eine Belüftungsnut, sondern mehrere an der Oberfläche der Otoplastik geführt werden, wie dies schematisch in Fig. 10 dargestellt ist. Die hohe Flexibilität der Nutauslegung führt dazu, dass je nach Applikationsbereich im Gehörgang gezielt unterschiedlich dimensionierte, bezüglich Cerumenschutz sowie akustischen Übertragungsverhältnissen jeweils optimierte Belüftungsnuten entlang der Otoplastik-Oberfläche realisiert werden können.

### 2b) Belüftungssysteme mit voll integrierten Kanälen

Diese Ausbildungsvariante der neuartigen Belüftungssysteme beruht auf mindestens abschnittsweise völlig in die Otoplastik integrierten, gegen die Gehörgangwandung geschlossenen Belüftungskanälen. Dieses System wird anschliessend anhand seiner Ausbildung an einer Otoplastik-Schale erläutert. Es ist aber zu betonen, dass dann, wenn an der betrachteten Otoplastik keine weiteren Aggregate zu integrieren sind und sie als Vollplastik ausgebildet ist, die nachfolgenden Ausführungen sich selbstverständlich auch auf eine Kanalführung beliebig durch die erwähnte Vollplastik hindurch beziehen.

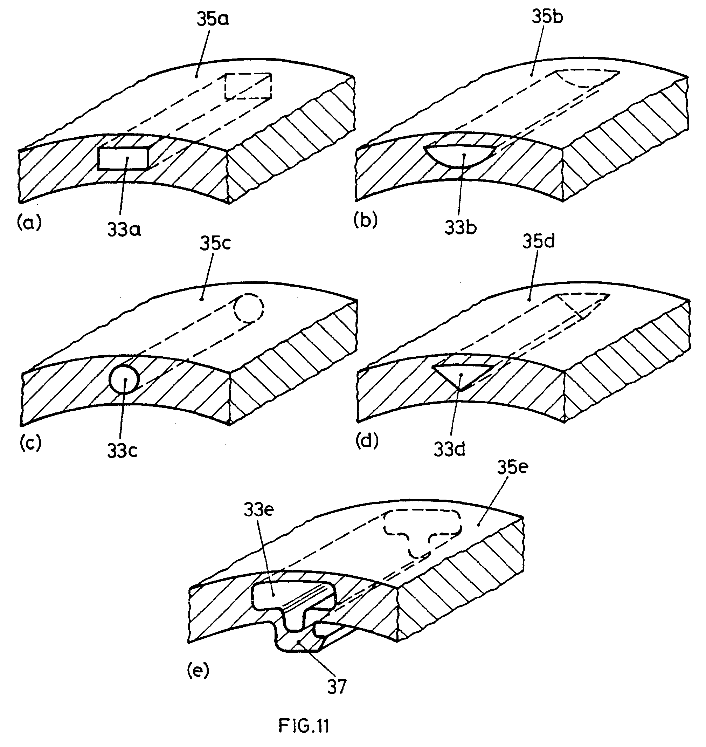

In Fig. 11 sind in Analogie zu Fig. 7 unterschiedliche Querschnittsformen und Flächenverhältnisse der vorgeschlagenen Belüftungskanäle 33a bis 33e dargestellt. Gemäss Fig. 11(a) hat der in die Otoplastikschale 35a eingebaute Belüftungskanal 33a Rechteck- oder Quadrat-Querschnittsform. Bei der Ausführungsform gemäss Fig. 11(b) hat er, 35b, eine Kreissektor- oder Ellipsensektor-förmige Kanalquerschnittsform. Bei der Ausführungsform gemäss Fig. 11(c) hat der vorgesehene Belüftungskanal 33c kreisförmige oder elliptische Querschnittsform, während er bei der Ausführungsvariante gemäss Fig. 11(d) eine dreieckförmige Querschnittform aufweist.

Bei der Ausführungsform gemäss Fig. 11(e) weist die Otoplastikschale eine komplexe Innenformung auf, z.B. eine daran integrierte Halterungspartie 37. Für optimale Platznutzung ist der hier vorgesehene Entlüftungskanal 35e mit einer Querschnittsform angelegt, die auch komplexe Formen der Otoplastikschale nutzt. Demnach erstreckt sich seine Querschnittsform kompliziert teilweise in die an die Schale 35e angebaute Halterungsleiste 37 hinein.

Rückblickend auf die Ausführungsvariante gemäss Abschnitt 2a) ist anzuführen, dass derartig komplexe, optimal den zur Verfügung stehenden Platz nutzende Querschnittsformen sich auch an gegen den Hörkanal offenen Belüftungsnuten realisieren lassen, ebenso, umgekehrt, Kanalführungen, wie sie für offene Nuten in den Fig. 9 und 10 dargestellt sind, an geschlossenen Belüftungskanälen.

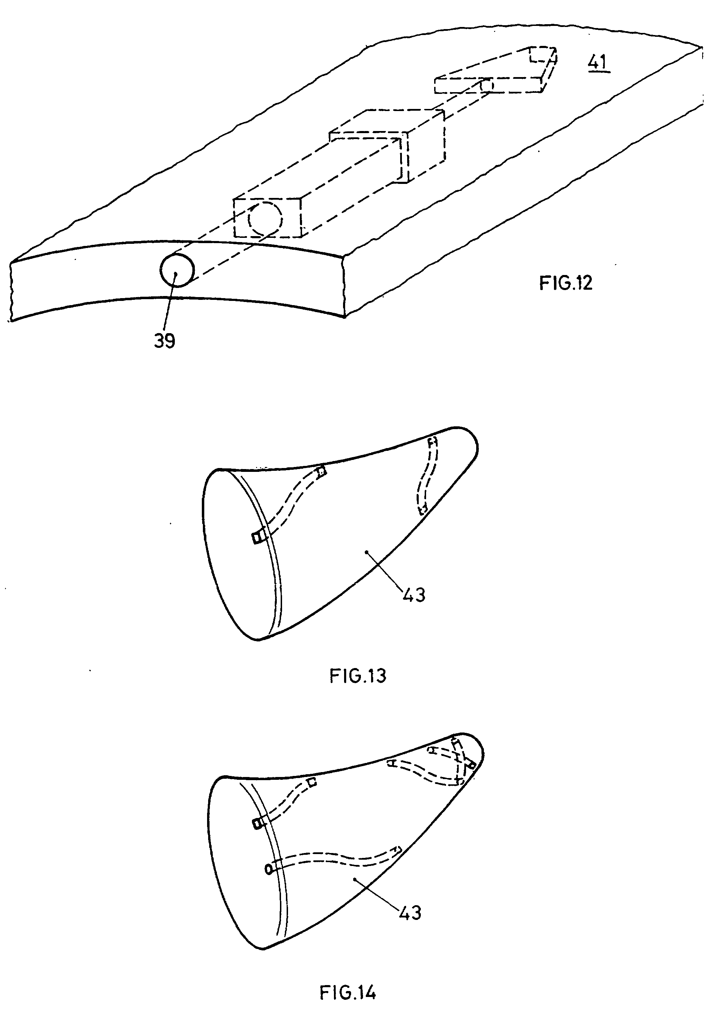

In Fig. 12 ist schliesslich eine Ausführungsvariante eines voll integrierten Belüftungskanals 39 dargestellt, der entlang seiner Längsausdehnung, wie dargestellt beispielsweise in der Otoplastikschale 41, unterschiedliche Querschnittsformen und/oder Querschnittsdimensionen aufweist, womit im Sinne der Realisation unterschiedlicher akustischer Impedanzelemente das akustische Übertragungsverhalten optimiert werden kann. In diesem Zusammenhang und auf den nachfolgenden Abschnitt 5) verweisend, kann auch darauf hingewiesen werden, dass wegen der Möglichkeit, komplexe akustische Impedanzverhältnisse zu realisieren, Belüftungskanäle, insbesondere der in diesem Abschnitt dargestellten geschlossenen Aufbauweise, durchaus mindestens abschnittsweise gleichzeitig als akustische Leiterabschnitte ausgangsseitig aktiver elektromechanischer Wandler, wie ausgangsseitig von Mikrophonen, beispielsweise bei Im-Ohr-Hörgeräten, ausgenützt werden können.

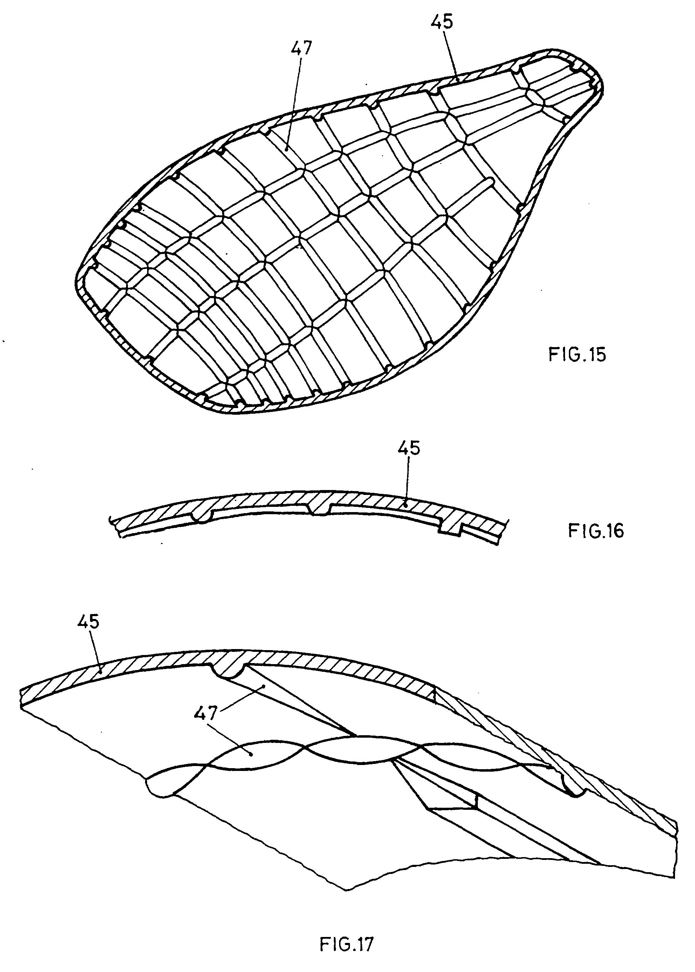

In den Fig. 13 und 14 ist in Analogie zu den Fig. 9 und 10 dargestellt, wie einerseits an der jeweiligen Otoplastik 43 die in diesem Abschnitt erläuterten integrierten Belüftungskanäle durch entsprechende Bahnführung verlängert bzw. anderseits wie zwei und mehr der erwähnten Kanäle, ggf. mit unterschiedlichen und/oder variierenden Kanalquerschnitten, in Analogie zu Fig. 12, an der Otoplastik integriert werden.

Durch die in den Abschnitten 2a) und 2b) dargestellten, auch beliebig kombinierbaren Möglichkeiten eröffnen sich dem Fachmann eine Unzahl Auslegungsvarianten der neuartigen Belüftungssysteme und insbesondere ein grosses Ausmass an Freiheit, aufgrund der verschiedenen, für sich dimensionierbaren Parameter, für die jeweilige individuelle Otoplastik optimalen Cerumenschutz und optimale akustische Übertragungsverhältnisse zu schaffen. Bei allen Ausführungsvarianten wird bevorzugterweise die spezifische individuelle Ausgestaltung des Systems berechnet bzw. rechnerisch modelliert, den erwähnten Bedürfnissen Rechnung tragend. Dann wird die individuelle Otoplastik realisiert. Wiederum eignet sich hierzu insbesondere das eingangs erläuterte Fertigungsverfahren mit additivem Aufbauprinzip, wie aus dem Prototypenbau bekannt, das dann mit dem optimierten Modellresultat gesteuert wird.

### 3. Formstabilitäts-optimierte Otoplastiken

In diesem Abschnitt geht es darum, neuartige Otoplastiken vorzustellen, welche optimal der Dynamik der Applikationsbereiche angepasst sind. Es ist beispielsweise bekannt, dass herkömmliche Im-Ohr-Otoplastiken der relativ grossen Gehörgangdynamik, z.B. beim Kauen, nicht Rechnung zu tragen vermögen, aufgrund ihrer im wesentlichen über alles gleichen Formstabilität. Desgleichen vermögen beispielsweise die akustischen Leiter zwischen Aussenohr-Hörgeräten und Gehörgang einer Dynamik des Applikationsbereiches nicht frei zu folgen. Bei Im-Ohr-Otoplastiken tritt dieselbe Problematik, teilweise abgeschwächt, auch bei Gehörschutz-Einrichtungen, Kopfhörern, Wasserschutzeinsätzen etc. auf.

Insbesondere wird dabei teilweise ihre intrinsische Funktion, beispielsweise Schutzwirkung, beeinträchtigt, wenn der erwähnten Applikationsbereichs-Dynamik zunehmend Rechnung getragen wird. Als Beispiel kann hierzu auf bekannte Gehörschutzeinrichtungen aus elastisch formveränderbaren Kunststoffen hingewiesen werden, die wohl der erwähnten Applikationsbereichs-Dynamik weitestgehend Rechnung tragen, dies aber auf Kosten ihres akustischen Übertragungsverhaltens.

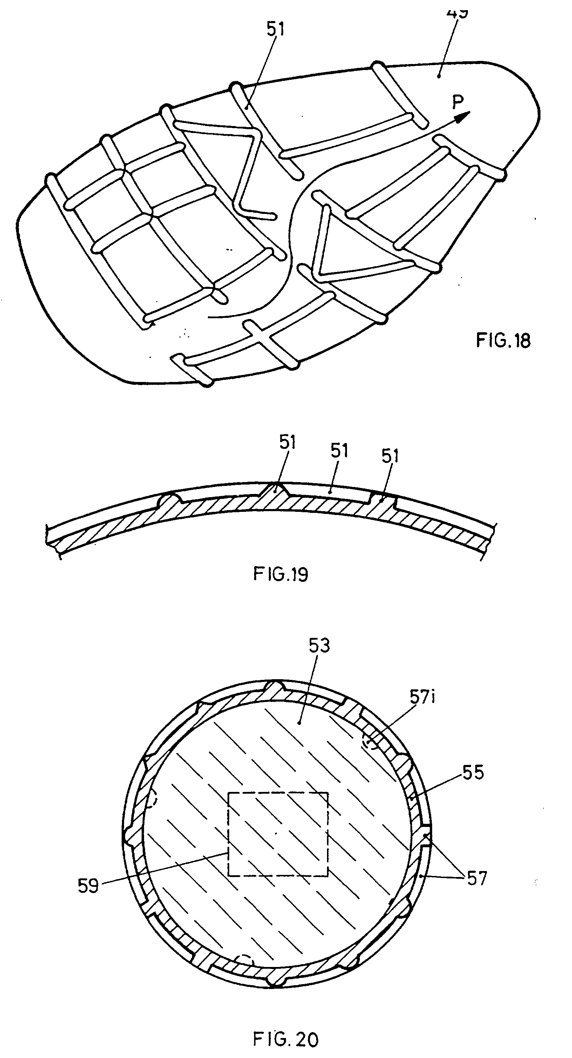

In Fig. 15 ist schematisch eine Längsschnittdarstellung einer Im-Ohr-Otoplastik wiedergegeben, in Fig. 16 eine schematische Querschnittsdarstellung eines Abschnitts dieser Otoplastik. Die Otoplastik - z.B. zur Aufnahme elektronischer Komponenten - weist eine Schale 45 auf, die strumpfartig, dünnwandig aus elastischem Material besteht. Die Formstabilität der - beim dargestellten Ausführungsbeispiel aussen glatten - Schalenhaut wird - wo erwünscht - durch an der Schale integral innen aufgesetzte Rippen 47 sichergestellt, die, bezüglich der Schalenhaut, aus dem gleichen Material gefertigt sind.

Je nach erforderlicher Dynamik der Im-Ohr-Otoplastik einerseits, um beispielsweise derjenigen des Gehörganges Rechnung zu tragen und den Anforderungen bezüglich der Abstützung und dem Schutz von Einbauten, wie bei einem Im-Ohr-Hörgerät, wird der Verlauf der Wandstärke von Schalenhaut 45, die Dichte und Gestalt der Rippen 47 vorgängig berechnet und danach die Otoplastik nach den berechneten Daten aufgebaut. Wiederum eignet sich hierzu das oben erläuterte Fertigungsverfahren unter Verwendung additiver Aufbauverfahren ausserordentlich gut. Selbstverständlich kann die eben erläuterte Ausbildung der Im-Ohr-Otoplastik durchaus kombiniert werden mit einem Belüftungssystem, wie es anhand der Figuren 7 bis 14 erläutert wurde. Insbesondere können die vorgesehenen Rippen zur Beeinflussung der Formstabilität bzw. Biegbarkeit in bestimmten Bereichen der Otoplastik auch mit unterschiedlichem Querschnittsprofil ausgebildet werden, ggf. auch in ihrer Längsausdehnung fortschreitend von einem Querschnitt zum andern übergehend.

In Form einer perspektivischen Darstellung ist in Fig. 17 rein beispielsweise die Ausbildung der Aussenhaut 45 mit Rippen 47 mit variierenden Querschnittsflächen entlang ihrer Längsausdehnung schematisch dargestellt.

Anstelle oder ergänzend zu der gezielten Wandverstärkung und gezielten Auslegung des Biege- bzw. Torsionsverhaltens, kurz des Formverhaltens der Im-Ohr-Otoplastik, kann, wie erwähnt, zusätzlich zur Innenrippenbemusterung, wie dies in den Fig. 17 und 18 dargestellt ist, auch eine Aussenrippenbemusterung vorgesehen werden. Gemäss den Fig. 18 und 19 wird hierzu, ggf. mit gebietsweise unterschiedlicher Dichte, Ausrichtung und Profilform, auf der Aussenfläche der Otoplastik 49 ein Muster von Rippen 51 aufgearbeitet.

Gemäss Fig. 19 kann dies für die hier betrachteten Otoplastiken mit Hohlraum eingesetzt werden, aber auch für Otoplastiken mit keinem Hohlraum, also beispielsweise mit keinen Elektronikkomponenten, z.B. für Hörschutzeinrichtungen bzw. Wasserschutzeinrichtungen. Eine solche Otoplastik ist in einer Querschnittsdarstellung schematisch in Fig. 20 dargestellt. Dabei ist der Innenraum 53 beispielsweise aus äusserst kompressiblem Absorptionsmaterial gefertigt und von einer formgebenden Hautschale 55 umgeben mit der Rippenmusterung 57. Dabei sind "Haut" 55 und die Rippenmusterung 57 gemeinsam integral gefertigt. Hierzu eignet sich wiederum das eingangs erläuterte Fertigungsverfahren unter Zuhilfenahme additiver Aufbauverfahren. Wie weit in naher Zukunft diese additiven Aufbauverfahren unter Wechsel der verarbeiteten Materialen an einem Werkstück realisierbar sind, bleibe dahingestellt. Sollte dies möglich werden, so ist die Bahn frei, beispielsweise am Ausführungsbeispiel gemäss Fig. 20 auch den Füllstoff 53 gleichzeitig mit der Schalenhaut 55 und den Rippen 57 in jeweiligen Aufbauschichten sequentiell aufzubauen.

Rückblickend insbesondere auf die Fig. 18 und 19 ist ersichtlich, dass mit Hilfe der Aussenrippenmuster gleichzeitig Belüftungskanäle bzw. -Freiräume gebildet werden können, wie dies rein schematisch und beispielsweise durch den Pfad P dargestellt ist.

Nochmals auf Fig. 20 zurückkommend, ist es durchaus möglich, falls erforderlich und wie in Fig. 20 gestrichelt bei 57ᵢ dargestellt ist, auch dann, wenn die Im-Ohr-Otoplastik materialgefüllt ist, also nicht zur Aufnahme weiterer Baueinheiten, wie von Elektronikbaueinheiten, bestimmt ist, an der Schalenhaut 55 ein Innenrippenmuster 57ᵢ vorzusehen.

Wie weiter in Fig. 20 gestrichelt bei 59 dargestellt ist, können auch Otoplastiken geschaffen werden, die wohl einen Hohlraum für aufzunehmende Aggregate wie Elektronikkomponenten freilassen, bei denen aber der Zwischenraum, zwischen einem solchen Hohlraum 59, spezifisch auf die notwendigen Volumina und Formen der zusätzlich einzubauenden Einheiten ausgelegt und die Schalenhaut 55 beispielsweise durch ein federndes oder schalldämmendes Material gefüllt ist oder einzubauende Komponenten mit einem solchen Material bis zur Schalenhaut 55 ausgegossen sind.

Die Schalenhaut 55 bzw. 45, gemäss den Figuren 15, 16 und 17, kann durchaus aus elektrisch leitendem Material gefertigt sein, womit gleichzeitig eine elektrische Abschirmwirkung für innenliegende Elektronikkomponenten geschaffen wird. Dies gilt auch ggf. für die Füllung 53 gemäss Fig. 20.

Anhand der Figuren 15 bis 20 wurde eine Otoplastik am Beispiel einer Im-Ohr-Otoplastik dargestellt, deren Schale mit innen- und/oder aussenliegenden Rippen formstabilisiert ist, was eine ausserordentlich leichte und gezielt formbare Bauweise ergibt. Selbstverständlich kann diese Bauweise falls erforderlich auch bei Aussenohr-Otoplastiken eingesetzt werden.

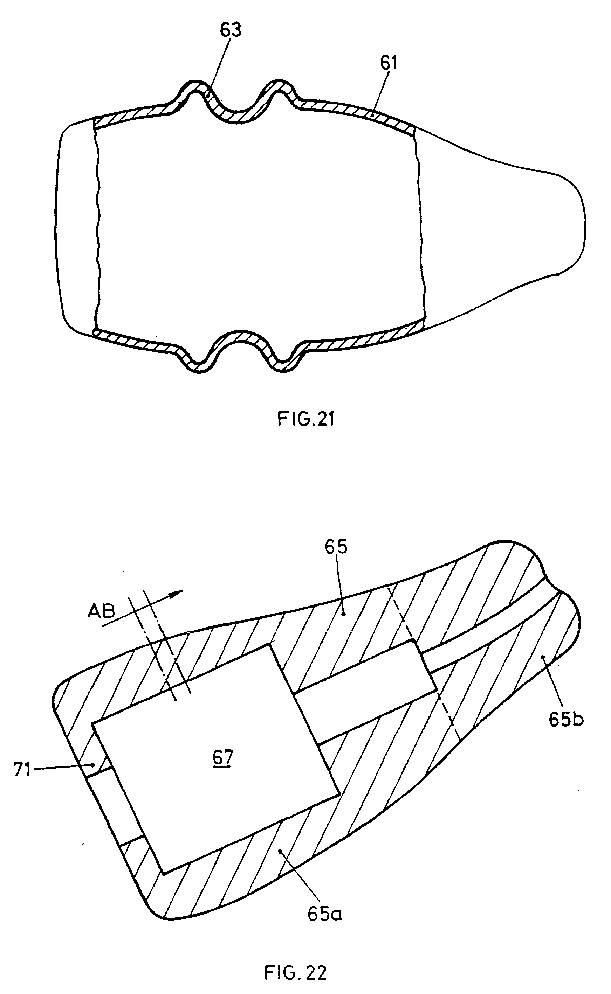

In Fig. 21 ist eine weitere Ausführungsvariante einer Im-Ohr-Otoplastik dargestellt, welche gezielt in einem Bereich biegbar bzw. stauchbar ist. Die Schale 61 einer Otoplastik, wie insbesondere die Schale eines Im-Ohr-Hörgerätes, weist hierzu in einem oder mehreren vorgegebenen Bereichen eine Wellen- bzw. Faltenschlauchausbildung 63 auf, woran sie, den jeweiligen Bedürfnissen entsprechend, bieg- bzw. stauchbar ist. Auch wenn Fig. 21 dieses Vorgehen anhand der Schale einer Im-Ohr-Otoplastik darstellt, lässt sich dieses Vorgehen durchaus und falls erforderlich auch für eine Aussenohr-Otoplastik realisieren. Wiederum wird hierzu bevorzugterweise das eingangs erläuterte Fertigungsverfahren eingesetzt.

Auch bei diesem Ausführungsbeispiel kann, wie dies anhand von Fig. 20 erläutert wurde, das Innenvolumen der Otoplastik mit den Erfordernissen entsprechendem Füllmaterial gefüllt werden bzw. können darin integrierte Einbauten in solchem Füllmaterial eingebettet werden, woraus eine höhere Stabilität des Gerätes resultiert und verbesserte Akustikverhältnisse.

### 4. Modulare Gehäuse/Einbauten

Insbesondere bei Im-Ohr-Hörgeräten besteht das Problem, dass der Applikationsbereich, d.h. der Gehörgang, seine Form ändert. Offensichtlich ist dies der Fall beim heranwachsenden Menschen. Aber auch bei Erwachsenen ändert sich der Gehörgang teilweise stark, meist in verengendem Sinne (z.B. sogenanntes Taucher-Ohr).

Bei Im-Ohr-Hörgeräten ergibt sich damit herkömmlicherweise das Problem, dass auch dann, wenn die Hörgeräteeinbauten an sich über lange Lebensabschnitte beibehalten werden könnten, beispielsweise lediglich das Übertragungsverhalten des Hörgerätes den jeweiligen Hörverhältnissen entsprechend nachgestellt werden müsste, trotzdem immer wieder neue Hörgeräte konzipiert werden müssen, lediglich aufgrund der Tatsache, dass die vormaligen nicht mehr zufriedenstellend in den Gehörgang passen.

Bereits die anhand von Abschnitt 3 erläuterten Massnahmen ergeben die Möglichkeit, dies zu verbessern, aufgrund der Tatsache, dass damit eine selbsttätige Formanpassung der Otoplastik an jeweilig sich ändernde Applikationsbereiche ermöglicht wird. In diesem Abschnitt sollen diesbezüglich weitere Massnahmen, insbesondere anhand von Im-Ohr-Otoplastiken, erläutert werden. Es ist aber darauf hinzuweisen, dass auch bei Aussenohr-Otoplastiken, wie Aussenohr-Hörgeräten, damit die Möglichkeit eröffnet wird, das "Gehäuse" zu wechseln, und zwar nicht nur, wenn dies vom Tragkomfort her notwendig wird, sondern auch, nach Wunsch, beispielsweise um das ästhetische Erscheinungsbild derartiger Aussenohr-Hörgeräte zu wechseln.

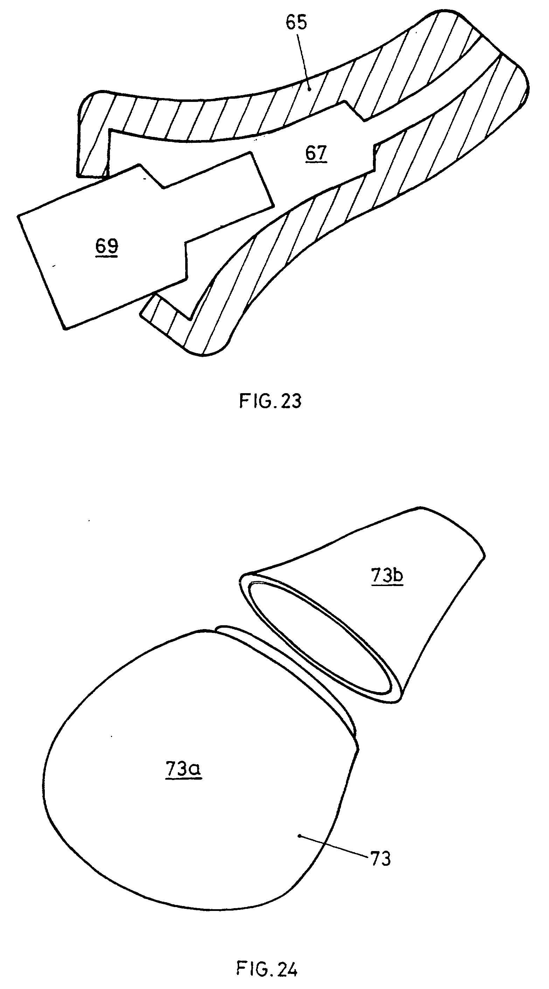

In Fig. 22 ist eine Im-Ohr-Otoplastik 65 schematisch und im Längsschnitt gezeigt, woran die Ausformung des Inneraumes 67 im wesentlichen der Form des in Fig. 23 schematisch dargestellten, aufzunehmenden Elektronikmoduls 69 entspricht. Die Otoplastik 65 besteht aus gummielastischem Material und kann, wie in Fig. 23 gezeigt, über das Elektronikmodul 69 gestülpt werden. Die Formung des Innenraumes 67 ist dergestalt, dass der oder ggf. die mehreren aufzunehmenden Module formschlüssig direkt durch die Otoplastik 65 positioniert und gehaltert werden. Aufgrund dieses Vorgehens ist es leicht möglich, ein und dieselben Elektronikmodule 69 mit unterschiedlichen Otoplastiken 65 zu versehen, um so beispielsweise bei einem heranwachsenden Kind der sich verändernden Gehörgangausbildung Rechnung zu tragen. Die Otoplastik wird für das Im-Ohr-Hörgerät praktisch zum leicht auswechselbaren Wegwerf-Accessoire. Nicht nur, um sich ändernden Verhältnissen am Applikationsbereich, nämlich dem Gehörgang, Rechnung zu tragen, sondern auch einfach aus Verschmutzungsgründen, kann die Otoplastik 65 leicht gewechselt werden. Dieses Konzept kann sogar dazu ausgenützt werden, ggf. - beispielsweise bei Gehörgangentzündungen - Medizinalapplikationen vorzunehmen, beispielsweise durch Applikation von Medikamenten an die Otoplastik-Aussenfläche oder mindestens, um in regelmässigen Abständen sterilisierte Otoplastiken einzusetzen.

Das anhand der Figuren 22 und 23 dargestellte Konzept lässt sich selbstverständlich mit den in den Abschnitten 2) und 3) dargelegten Konzepten kombinieren, und es wird bevorzugterweise die Otoplastik 65 nach dem in Abschnitt 1) erläuterten Fertigungsverfahren hergestellt, welches die Ausbildung komplexester Innenformen zur spiel- und vibrationsfreien Aufnahme des Moduls 69 ermöglicht.

Wie aus den Fig. 22 und 23 ersichtlich, wird beispielsweise als Teil der Modulhalterung die sonst bei herkömmlichen Im-Ohr-Hörgeräten vorgesehene Phaseplate 1 integral mit der Otoplastik gebaut. Dasselbe gilt für weitere Halterungen und Aufnahmen für Elektronikkomponenten des Hörgerätes. Realisiert man das unter Abschnitt 1) dargelegte Schicht-um-Schicht-Aufbauverfahren, wie in Fig. 22 strichpunktiert und in der mit dem Pfeil AB angedeuten Richtung, so dürfte es ohne weiteres möglich sein, die Otoplastik in der erwähnten Aufbaurichtung AB je nach Erfordernissen in den jeweiligen Bereichen aus unterschiedlichen Materialien zu fertigen. Dies gilt auch für die in den Abschnitten 2) und 3) dargelegten Otoplastiken sowie für die in den folgenden Abschnitten 5), 6) und 7) erläuterten. Am Beispiel von Fig. 22 ist es somit durchaus möglich, den Bereich 65ₐ aus gummielastischem Material zu fertigen, hingegen den Ausgangsbereich 65_{b} aus formstabilerem Material.

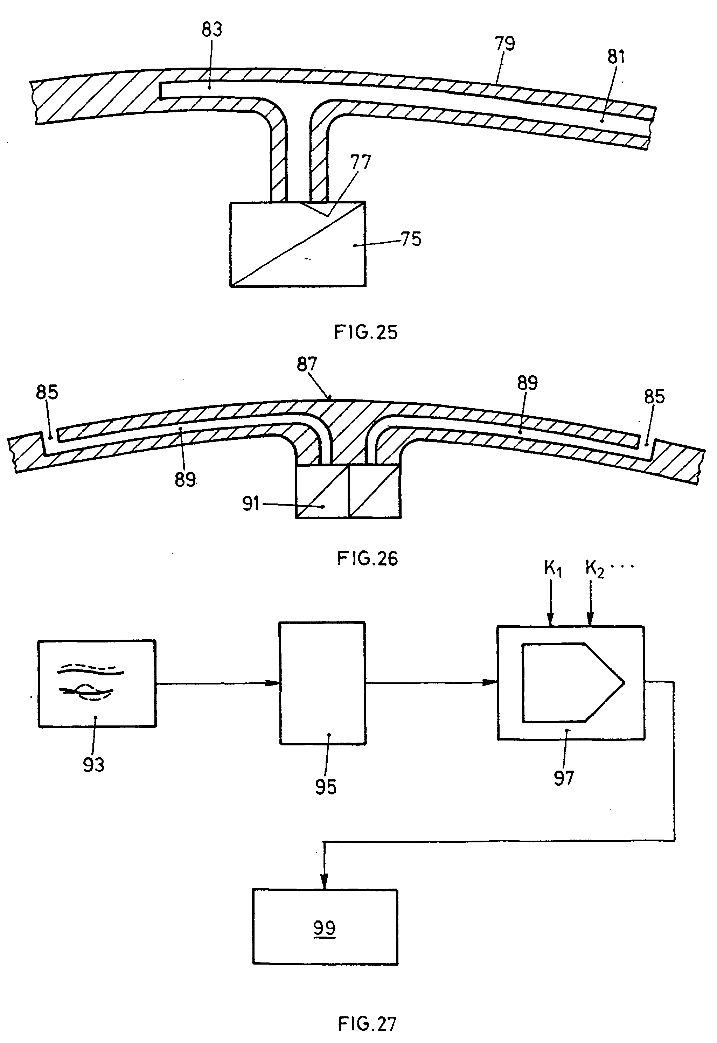

In Fig. 24 ist eine weitere Ausführungsform einer Otoplastik, wiederum als Beispiel anhand eines Im-Ohr-Hörgeräts, dargestellt, welche ein einfaches, rasches Auswechseln der inneren Einbauten ermöglicht. Grundsätzlich wird dabei vorgeschlagen, an einer Im-Ohr-Otoplastik mit Einbauten die Otoplastik-Schale mehrteilig und assemblierbar auszubilden, wie dies Fig. 24 zeigt. Mittels schnell betätigbaren Verschlüssen, wie Einrastverschlüssen, Einklinkverschlüssen oder gar bajonettähnlichen Verschlüssen, wird ermöglicht, an der Im-Ohr-Otoplastik Gehäuseteile 73a und 73b rasch voneinander zu trennen, die Einbauten wie Elektronikmodule daraus zu entfernen und sie in eine neue Schale wieder einzubauen, ggf. mit geänderter Aussenformung oder grundsätzlich in eine neue Schale, auch wenn dies beispielsweise aus Reinigungsgründen, Sterilitätsgründen etc. erforderlich ist. Wird dabei vorgesehen, die bereits gebrauchte Schale wegzuwerfen, ist es ohne weiteres möglich, die Verbindungen der Schalenteile so auszubilden, dass die Schale nur zerstörend geöffnet werden kann, beispielsweise indem von aussen nicht zugängliche Verriegelungsorgane wie Klinken vorgesehen werden und die Schale für deren Entfernung aufgeschnitten wird.

Auch diese Ausführungsform kann selbstverständlich mit den bis anhin beschriebenen und noch zu beschreibenden Ausführungsvarianten kombiniert werden.

### 5. Integration akustischer Leiter in Otoplastiken bzw. deren Schalen

Bei Aussenohr- wie auch bei Im-Ohr-Hörgeräten ist es üblich, vorgesehene akustisch/elektrische Wandler oder elektro-akustische Ausgangswandler eingangs- bzw. ausgangsseitig über als eigenständige Teile assemblierte akustische Leiter, nämlich röhrchenähnliche Gebilde, mit der Umgebung des Hörgerätes zu koppeln, oder aber, insbesondere bei eingangsseitigen akustisch/elektrischen Wandlern, diese mit ihrer Aufnahmefläche unmittelbar im Bereiche der Oberflächen des Hörgerätes zu platzieren, ggf. lediglich durch geringfügige Hohlräume und Schutzvorkehrungen von der Umgebung getrennt.

Dabei besteht bei der Konzeption derartiger Hörgeräte eine relativ grosse Bindung, wo im Hörgerät die eigentlichen Wandler und wo am Hörgerät die eigentlichen Kopplungsöffnungen zur Umgebung vorzusehen sind. Es wäre höchst wünschbar, bezüglich der Anordnung von Kopplungsöffnungen zur Umgebung und Anordnung der erwähnten Wandler innerhalb des Hörgerätes grösstmögliche Konzeptionsfreiheit zu haben.

Dies wird grundsätzlich dadurch erreicht, dass die erwähnten akustischen Leiter - eingangsseitig von akustisch/elektrischen Wandlern bzw. ausgangsseitig von elektrisch/akustischen Wandlern - in die Otoplastik bzw. in die Wandung von Otoplastikschalen integriert werden.

In Fig. 25 ist dies rein schematisch dargestellt. Ein Wandlermodul 75 weist einen akustischen Ein- bzw. Ausgang 77 auf. Die Schale 79 der Otoplastik eines Im-Ohr- oder eines Aussenohr-Hörgerätes oder eines Kopfhörers weist, in ihr integriert, einen akustischen Leiter 81 auf. Er liegt mindestens abschnittsweise und wie in Fig. 25 dargestellt innerhalb der Wandung der Otoplastikschale 79. Mittels akustischer Stichleitungen bzw. Leitungsabschnitten 83 wird vorzugsweise die jeweilige akustische Impedanz des akustischen Leiters 81 angepasst. Dieses Konzept, mit Blick auf Aussenohr-Hörgeräte, ermöglicht es, entlang des Hörgerätes versetzt und wo erwünscht akustische Eingangsöffnungen 85 vorzusehen, diese über in der Otoplastik bzw. deren Schale 87 integrierte akustische Leiter 89 an die vorgesehenen akustisch/elektrischen Wandler 91 anzukoppeln, im wesentlich unabhängig davon, wo diese Wandler 91 im Hörgerät eingebaut werden. So ist in Fig. 26 nur beispielsweise dargestellt, zwei Wandler zu einem Modul zu zentralisieren und ihre Eingänge mit den erwünschten Aufnahmeöffnungen 85 durch die erwähnte Führung der akustischen Leiter 89 zu verbinden. Aus Betrachtung der Figuren 25 und 26 und den Ausführungen in Abschnitt 2) betreffs der neuartigen Belüftungssysteme wird ersichtlich, dass es durchaus möglich wird, Belüftungskanäle auch als akustische Leiterkanäle zu nutzen, insbesondere wenn dabei, wie in Fig. 25 schematisiert, mittels akustischer Anpassglieder 83 die akustischen Impedanzverhältnisse gezielt ausgelegt werden.

### 6. Kennzeichnung von Otoplastiken

Bei der Fertigung von Otoplastiken, insbesondere von Im-Ohr-Otoplastiken, wird jede individuell für deren jeweiligen Träger angepasst. Deshalb wäre es äusserst erwünscht, jede gefertigte Otoplastik, wie erwähnt insbesondere jede Im-Ohr-Otoplastik, dabei ganz besonders jedes Im-Ohr-Hörgerät, zu kennzeichnen. Es wird deshalb vorgeschlagen, in die Otoplastik hinein bzw. in deren Schale, durch Einkerbungen und/oder durch Auswölbungen eine individuelle Kennzeichnung vorzusehen, welche nebst dem individuellen Besteller - z.B. Hersteller - Produktserienummer, Links-Rechtsapplikation etc. enthalten kann. Eine solche Kennzeichnung wird in weitaus bevorzugter Art und Weise bei der Fertigung der Otoplastik mit dem unter 1) beschriebenen Abtragverfahren erstellt. Damit wird sichergestellt, dass ab der Fertigung jegliche Verwechslung der Otoplastiken ausgeschlossen ist. Insbesondere wichtig ist dies bei der nachfolgenden, ggf. automatisierten Assemblierung mit weiteren Modulen, so beispielsweise der Assemblierung von Im-Ohr-Hörgeräten.

Dieses Vorgehen kann selbstverständlich kombiniert mit einem oder mehreren der unter den Abschnitten 2) bis 5) beschriebenen Aspekten realisiert werden.

### 7. Optimierung von Otoplastiken bezüglich der Dynamik des Applikationsbereiches

Für die Formnahme von Otoplastiken für die Im-Ohr-Applikation, so beispielsweise für Im-Ohr-Hörgeräte, ist es heute üblich, vom Gehörgang, beispielsweise in Silikon, einen Abdruck zu nehmen. Berücksichtigt man nun die relativ grosse Bewegungsdynamik des Gehörganges, beispielsweise beim Kauvorgang, so ist ersichtlich, dass die Abstützung der Im-Ohr-Otoplastikform auf einen praktisch einer Momentaufnahme entsprechenden Abdruck kaum zu einem Resultat führt, das im Gebrauch völlig zu befriedigen vermag. Wie dies nun in Fig. 27 anhand eines vereinfachten Funktionsblock/Signalflussdiagrammes dargestellt ist, wird vom dynamischen Applikationsgebiet, dargestellt durch den Block 93, an mehreren der in der Praxis erfolgenden Dynamik entsprechenden Positionen Form genommen bzw., filmähnlich, die Dynamik des Applikationsbereiches an sich registriert. Die resultierenden Datensätze werden in einer Speichereinheit 95 abgelegt. Auch bei herkömmlichem Vorgehen durch Abdrucknahme kann dies durchaus realisiert werden, indem vom Applikationsbereich in zwei oder mehr Positionen die der praktischen Dynamik entsprechenden Abdrücke genommen werden.

Es werden anschliessend diese Abdrücke abgetastet und die jeweiligen digitalen Datensätze in die Speichereinheit 95 abgelegt. Als weitere Möglichkeit kann beispielsweise die Dynamik des Applikationsbereiches durch Röntgenaufnahmen erfasst werden.

Es werden mithin je nach zu erzielender Genauigkeit mehrere "Bilder" oder gar praktisch ein "Film" des Bewegungsmusters vom interessierenden Applikationsbereich registriert. Die in der Speichereinheit 95 registrierten Daten werden anschliessend einer Recheneinheit 97 zugeführt. Ausgangsseitig steuert die Recheneinheit 97 den Fertigungsprozess 99 für die Otoplastik. Werden z.B., und wie bis heute üblich, Im-Ohr-Otoplastiken gefertigt mit relativ harter Schale, so berechnet die Recheneinheit 97 aus den an der Speichereinheit 95 abgelegten Dynamikdaten und ggf., wie bei K schematisch dargestellt, weiteren Fertigungsparametern, die beste Passform für die Otoplastik, damit optimaler Tragkomfortfort im Alltag erzielt wird, bei Erhalt ihrer Funktionalität. Wird die zu fertigende Otoplastik nach dem in Abschnitt 3) dargelegten Prinzip realisiert, so wird an der Recheneinheit 97 ermittelt, welche Otoplastikbereiche wie zu gestalten sind bezüglich ihrer Flexibilität, Biegbarkeit, Stauchbarkeit etc. Ausgangsseitig steuert, wie erwähnt, die Recheneinheit 97 den Fertigungsprozess 99, bevorzugterweise dabei den Fertigungsprozess, wie er im Abschnitt 1) als bevorzugter Prozess dargelegt wurde.

## Patentansprüche

1. Otoplastik mit einer eine Aussenfläche bildenden Schale aus einem Material und mit Kennzeichnungs-Einund/oder -Ausbuchtungen, **dadurch gekennzeichnet, dass** die Ein- und/oder Ausbuchtungen im Material der Schale vorhanden sind und ihre Oberfläche gleich beschaffen ist wie angrenzende Oberflächen-Bereiche der Schale.

2. Otoplastik nach Anspruch 1, **dadurch gekennzeichnet, dass** die Ein- und/oder Ausbuchtungen eine jeweilige Schale individualisieren.

3. Otoplastik nach einem der Ansprüche 1 oder 2, **dadurch gekennzeichnet, dass** die Oberfläche der Einbuchtungen und/oder Ausbuchtungen mindestens teilweise und mindestens zum Teil mit einem weiteren Material belegt sind, vorzugsweise mit einer Farbe oder einem Lack.

4. Otoplastik nach einem der Ansprüche 1 bis 3, **dadurch gekennzeichnet, dass** sie ein Hörgerät ist.

5. Verfahren zur Herstellung von Otoplastiken, **dadurch gekennzeichnet, dass**
• eine Schale gefertigt wird, die eine Aussenfläche bildet und die aus einem Material besteht,
• auf einer Oberfläche der Schale ein Kennzeichnungs-Ein- und/oder Ausbuchtungsmuster vorgesehen wird,
• eine nachfolgende individualisierte Fertigung der Otoplastik mittels der Kennzeichnungs-Ein- und/oder Ausbuchtungen gesteuert wird.

6. Verfahren nach Anspruch 5, **dadurch gekennzeichnet, dass** die Fertigung mindestens zum Teil durch maschinelles Erkennen des Kennzeichnungs-Ein- und/oder Ausbuchtungsmusters automatisiert wird.

7. Verwendung des Verfahrens nach einem der Ansprüche 5 oder 6 zur Fertigung von Hörgeräten.

## Claims

1. Otoplasty with a shell made of one material, whereby the shell has an outer surface, and with identifying indentations and/or embossments, **characterized in that** the indentations and/or embossments are present in the material of the shell and their surface is of the same nature as adjacent surface regions of the shell.

2. Otoplasty according to Claim 1, **characterized in that** the indentations and/or embossments individualize a respective shell.

3. Otoplasty according to either of Claims 1 and 2, **characterized in that** the surface of the indentations and/or embossments is at least partly and at least in part coated with a further material, preferably with a paint or a varnish.

4. Otoplasty according to one of Claims 1 to 3, **characterized in that** it is a hearing device.

5. Method for producing otoplasties, **characterized in that**
• a shell which forms an outer surface and consists of one material is produced,
• a pattern of identifying indentations and/or embossments is provided on a surface of the shell,
• subsequent individualized production of the otoplasty is controlled by means of the identifying indentations and/or embossments.

6. Method according to Claim 5, **characterized in that** the production is automated at least in part by machine detection of the pattern of identifying indentations and/or embossments.

7. Use of the method according to either of Claims 5 and 6 for producing hearing devices.

## Revendications

1. Otoplastique avec une coque formant une face extérieure en un matériau et avec des creux et/ou bombements de caractérisation, **caractérisé en ce que** les creux et/ou bombements sont présents dans le matériau de la coque et leur surface est de la même constitution que les zones de surface avoisinantes de la coque.

2. Otoplastique selon la revendication 1, **caractérisé en ce que** les creux et/ou bombements individualisent une coque respective.

3. Otoplastique selon l'une des revendications 1 ou 2, **caractérisé en ce que** la surface des creux et/ou bombements est recouverte au moins partiellement et au moins en partie par un autre matériau, de préférence d'une couleur ou d'un vernis.

4. Otoplastique selon l'une des revendications 1 à 3, **caractérisé en ce qu'**il est un appareil auditif.

5. Procédé de fabrication d'otoplastiques, **caractérisé en ce que**:
• une coque est fabriquée qui forme une face extérieure et qui est constituée d'un matériau,
• sur une surface de la coque, un dessin de creux et/ou de bombements de caractérisation est prévu,
• une fabrication individualisée suivante de l'otoplastique est commandée au moyen des creux et/ou bombements de caractérisation.

6. Procédé selon la revendication 5, **caractérisé en ce que** la fabrication est automatisée au moins en partie par une détection par machine du dessin de creux et/ou de bombements de caractérisation.

7. Utilisation du procédé selon l'une des revendications 5 ou 6 pour la fabrication d'appareils auditifs.
